# FASCICULE DE BREVET EUROPEEN

(11) **EP 1 082 082 B1**
(45) Date de publication et mention de la délivrance du brevet: **10.12.2003**
(21) Numéro de dépôt: 00911018.0
(22) Date de dépôt: 21.03.2000
(51) Int. Cl.: A61K 7/02

(54) **COMPOSITION CONTENANT UN ACTIF INSTABLE EN MILIEU OXYDANT, ET SES UTILISATIONS NOTAMMENT COSMETIQUES**
ZUBEREITUNG ENTHALTEND EINEN LABILEN WIRKSTOFF IN EINER OXIDIERENDEN UMGEBUNG UND IHRE ANWENDUNG INSBESONDERE IN DE KOSMETIK
COMPOSITION CONTAINING AN UNSTABLE ACTIVE AGENT IN AN OXIDIZING MEDIUM AND USES THEREOF, ESPECIALLY COSMETIC USES

(30) Priorité: 31.03.1999 FR 9904046
(43) Date de publication de la demande: 14.03.2001
(73) Titulaire: L'OREAL, 75008 Paris (FR)
(72) Inventeur: JAGER-LEZER, Nathalie, F-92340 Bourg la Reine (FR); LORANT, Raluca, F-94320 Thiais (FR)
(74) Mandataire: Rasson, Catherine
(86) Numéro de dépôt international: FR0000705
(87) Numéro de publication internationale: WO00057844

(56) Documents cités:
- EP-A- 0 295 886
- EP-A- 0 501 791
- EP-A- 0 706 789
- EP-A- 0 893 467
- US-A- 5 599 533
- US-A- 5 853 741
- PATENT ABSTRACTS OF JAPAN vol. 1999, no. 04, 30 avril 1999 (1999-04-30) & JP 11 021227 A (KOSE CORP), 26 janvier 1999 (1999-01-26)

## Description

L'invention se rapporte à une composition contenant un actif instable en milieu oxydant, stabilisé, à l'utilisation de la dite composition notamment dans le domaine cosmétique, et à un procédé de traitement de la peau humaine à l'aide d'une telle composition.

L'invention se rapporte encore à l'utilisation d'un organopolysiloxane élastomère réticulé à groupement oxyalkyléné, en vue de stabiliser un actif instable en milieu oxydant.

Il est connu d'introduire dans les compositions cosmétiques et/ou dermatologiques des actifs en vue d'apporter des traitements spécifiques à la peau et/ou aux cheveux, par exemple pour nettoyer la peau, pour lutter contre le dessèchement, le vieillissement ou la pigmentation de la peau, pour traiter l'acné ou certains désordres de la peau (eczéma, psoriasis), pour combattre les surcharges pondérales, pour favoriser la restructuration de la peau ou son renouvellement cellulaire, pour traiter la séborrhée des cheveux.

Par exemple, on cherche depuis longtemps à formuler l'acide ascorbique (ou vitamine C) et le rétinol (vitamine A) dans les domaines cosmétique et dermatologique, sous différentes formes galéniques, du fait de leurs nombreuses propriétés bénéfiques. En particulier, l'acide ascorbique stimule la synthèse du tissu conjonctif et notamment du collagène, renforce les défenses du tissu cutané contre les agressions extérieures telles que les rayonnements ultraviolets et la pollution, compense la déficience en vitamine E de la peau, dépigmente la peau et possède une fonction anti-radicaux libres. Du fait de ses propriétés, l'acide ascorbique est efficace pour nettoyer la peau et également pour lutter contre les signes du vieillissement de la peau, par exemple pour améliorer l'éclat du teint et estomper les ridules et/ou rides de la peau.

Par ailleurs, les effets du rétinol sur la différenciation cellulaire permettent d'envisager, entre autres, son utilisation pour lutter efficacement contre l'apparition des rides et ridules, contre la sécheresse cutanée, la rugosité et/ou la rigidité de la peau. Une application répétée de compositions cosmétiques contenant du rétinol permet, entre autre, d'effacer les rides, de lisser la peau, de réparer les petites déchirures de l'épiderme.

Malheureusement, ces actifs sont instables en milieu oxydant et donc très sensibles à certains paramètres de l'environnement comme par exemple la lumière, l'oxygène et l'eau. Il s'ensuit donc une dégradation rapide de ces actifs lorsqu'ils sont en contact avec notamment un de ces paramètres, ce qui va à l'encontre de l'efficacité recherchée.

Dans l'état de la technique ce problème a été diversement traité. Ainsi, pour diminuer ou retarder la dégradation de l'acide ascorbique en solution, le document US-A-5,140,043 préconise de le stabiliser en l'introduisant dans des solutions hydroalcooliques, formées d'au moins 80 % d'eau et ayant un pH inférieur à 3,5. En raison de la forte acidité de ces solutions, leur utilisation dans le domaine cosmétique est difficilement envisageable. En effet, une application répétée de ces solutions peut perturber l'équilibre de la peau et en particulier irriter, voire brûler la peau.

D'autres modes de stabilisation de l'acide ascorbique ont été envisagés notamment par enrobage (technique décrite dans le document FR-A-1600826) ou par granulation de l'acide ascorbique (technique illustrée dans le document JP-A-53-127819, pour l'agro-alimentaire). Mais ces techniques sont d'une part coûteuses et peuvent d'autre part altérer l'acide ascorbique, par exemple lors d'un chauffage, et/ou conduire à des compositions peu cosmétiques, comme c'est le cas des granulés.

En outre, le document US-A-5,853,741 décrit la stabilisation de l'acide ascorbique par des silicones élastomères non-émulsifiants connus sous les références commerciales Gransils de la société Grand Industrie. L'inconvénient de ces produits est d'apporter un effet huileux, gras, sans effet frais, ce qui ne permet pas ou difficilement leur utilisation en milieu chaud et humide et/ou par les utilisateurs à peaux grasses. De plus ces polymères sont totalement hydrofuges et difficilement incorporables en phase aqueuse. Du fait de leur forte incompatibilité avec l'eau et en particulier avec la sueur, cette dernière n'est pas absorbée par ces polymères et a même tendance à « perler » à la surface de la peau, lorsque celle-ci transpire.

La stabilisation du rétinol a aussi fait l'objet de nombreuses recherches. Ainsi, le document WO-A-93/00085 décrit des émulsions E/H comprenant du rétinol et un système stabilisant constitué d'un agent chélatant et un anti-oxydant. On connaît aussi par le document EP-A-209509 l'utilisation de certains composés polyaminés comme anti-oxydants. Toutefois, aucun des composés de l'art antérieur ne permet d'obtenir une stabilisation satisfaisante du rétinol.

Il subsiste donc le besoin d'une composition utilisable notamment dans le domaine cosmétique, dans laquelle un actif instable en milieu oxydant soit stabilisé, qui soit confortable lors de l'application et qui ne provoque aucune irritation de la peau après application.

La demanderesse a maintenant trouvé une composition surmontant les inconvénients de l'art antérieur.

La présente invention a pour objet une composition contenant dans un milieu physiologiquement acceptable, au moins un actif instable en milieu oxydant et une phase huileuse comprenant des particules d'un organopolysiloxane solide élastomère réticulé comportant au moins un groupement oxyalkyléné, la dite composition n'étant pas une émulsion triple H/E/H ou E/H/E.

Grâce à la présence des particules d'organopolysiloxane élastomère à groupement oxyalkyléné dans la composition de l'invention, les actifs instables en milieu oxydant,. tels que l'acide ascorbique et le rétinol, présentent une meilleure stabilité, et donc une meilleure efficacité que dans une composition n'en contenant pas.

Aussi, l'invention se rapporte à l'utilisation de particules d'un organopolysiloxane solide élastomère réticulé comportant au moins un groupement oxyalkyléné, pour la stabilisation d'un actif instable en milieu oxydant.

On entend par milieu physiologiquement acceptable dans la composition de l'invention, un milieu non toxique et susceptible d'être appliqué sur la peau (y compris l'intérieur des paupières) ou les lèvres d'êtres humains.

Par « solide élastomère » on entend un matériau souple, déformable ayant des propriétés viscoélastiques et notamment la consistance d'une éponge ou d'une sphère souple. Son module d'élasticité est tel que ce matériau résiste à la déformation et possède une capacité limitée à l'extension et à la contraction. Ce matériau est capable de retrouver sa forme originelle suite à un étirement. Cet élastomère est formé de chaînes polymériques de haut poids moléculaire dont la mobilité est limitée par un réseau uniforme de points de réticulation.

Les organopolysiloxanes de la composition de l'invention contiennent un ou plusieurs groupements oxyalkylénés et en particulier oxyéthylénés (OE), par exemple de 1 à 40 motifs oxyalkylénés, de préférence de 1 à 20 et mieux de 10 à 20 motifs oxyalkylénés, pouvant former des chaînes polyoxyalkylènes et notamment polyoxyéthylénes. Ces groupements peuvent être pendants, en bout de chaîne ou destinés à lier deux parties de la structure siliconée. Les atomes de silicium portant ces groupements sont avantageusement au nombre d'environ 1 à 10 et mieux de 1 à 6.

Bien que l'invention concerne plus spécialement les organopolysiloxanes à groupement(s) oxyéthyléné(s) (à savoir les groupements ne comportant que des groupements oxyéthylénés comme groupements oxyalkylénés), elle peut aussi concerner les organopolysiloxanes à groupement(s) oxypropyléné(s), c'est-à-dire ne comportant que des groupements oxypropylénés comme groupements oxyalkylénés. Les organopolysiloxanes peuvent aussi comporter à la fois un ou plusieurs groupement(s) oxyéthyléné(s), 1 à 20 (OE) par exemple, et un ou plusieurs groupement(s) oxypropyléné(s) (OP), 0 à 20 par exemple ; ces organopolysiloxanes sont appelés aussi des organopolysiloxanes à groupement(s) alkyléthoxy-propyléné(s). De préférence, le nombre de groupements oxyéthylénés est supérieur au nombre de groupements oxypropylénés.

Par ailleurs, la structure siliconée formant le squelette polymérique de l'organopolysiloxane à groupement(s) oxyalkyléné(s) est avantageusement une structure polydiméthylsiloxane (PDMS) dont éventuellement une partie des groupes méthyle est substituée par des groupements alkyle en C₂ à C₃₀ et de préférence en C₈ à C₂₄, et mieux de C₁₀ à C₂₀ ou phényle, soit en bout de chaîne soit pendants.

Par ailleurs, l'organopolysiloxane à groupement(s) oxyalkyléné(s) peut comporter un ou plusieurs squelette(s) siliconé(s) relié(s) entre eux par un ou plusieurs groupements oxyalkylénés et de préférence oxyéthylénés tels que définis précédemment ou par un ou plusieurs groupements alkylénés, le nombre de groupement alkyléné allant de 1 à 20 et de préférence de 1 à 10. De préférence, il comporte au moins deux squelettes polymériques liés entre eux. Avantageusement, le ou les squelettes siliconés des organopolysiloxanes de la composition selon l'invention comportent de 26 à 80 atomes de silicium.

Les organopolysiloxanes élastomères de la composition de l'invention présentent un remarquable pouvoir d'épaississement d'une phase huileuse et d'émulsification d'une phase huileuse dans une phase aqueuse et vice versa ; ils gonflent dans la phase huileuse. Ils ne sont pas desséchants pour la peau et apportent de bonnes propriétés cosmétiques, notamment de douceur, de fraîcheur et de matité. Ces élastomères conduisent à des compositions confortables à l'application, de bon étalement, douces et non collantes au toucher. Ces propriétés cosmétiques sont dues, d'une part à la texture des organopolysiloxanes et, d'autre part à leurs propriétés comparables à celles de microéponges piégeant les milieux huileux et en particulier ceux de la composition et ceux sécrétés par la peau.

Les organopolysiloxanes élastomères utilisés dans la composition conforme à l'invention sont partiellement ou totalement réticulés et de structure tridimensionnelle. Inclus dans une phase huileuse, ils se transforment, selon le taux de phase huileuse utilisé, d'un produit d'aspect spongieux lorsqu'ils sont utilisés en présence de faibles teneurs en phase huileuse en un gel homogène, en présence de quantités de phase huileuse plus élevées. La gélification de la phase huileuse par ces élastomères peut être totale ou partielle.

Les élastomères de l'invention se présentent sous forme de poudre ou de gel contenant un organopolysiloxane élastomère de structure tridimensionnelle dispersé dans une phase huileuse. Cette phase huileuse, appelée encore phase grasse liquide, peut comprendre tout corps non aqueux ou mélange de corps non aqueux, liquide à température ambiante (25°C) et à la pression atmosphérique (760 mm de Hg).

Les organopolysiloxanes élastomères utilisés selon l'invention peuvent être choisis parmi les polymères réticulés obtenus par réaction d'addition et de réticulation en milieu non aqueux, en présence d'un catalyseur notamment du type platine, d'au moins :
- (a) un premier organopolysiloxane (i) ayant au moins deux groupes vinyliques en position α-ω de la chaîne siliconée ; et
- (b) un second organopolysiloxane (ii) ayant au moins un atome d'hydrogène lié à un atome de silicium par molécule et au moins un groupement oxyalkyléné notamment oxyéthyléné.

En particulier, l'organopolysiloxane (i) est choisi parmi les polydiméthylsiloxanes (PDMS) et est plus spécifiquement un α-ω-diméthylvinyl polydiméthylsiloxane.

L'organopolysiloxane (ii) est choisi notamment parmi les polydiméthylsiloxanes comportant un ou plusieurs atome(s) d'hydrogène lié chacun à un atome de silicium, et un ou plusieurs groupements oxyéthylénés et éventuellement un ou plusieurs groupements oxypropylénés, liés à un atome de silicium via un radical alkylène ayant de 1 à 22 atomes de carbone.

Eventuellement les chaînes silicones des premiers et seconds organopolysiloxanes (i) et (ii) comportent des chaînes pendantes alkyle en C₁ à C₆ et/ou des chaînes aryle.

Les organopolysiloxanes élastomères de la composition selon l'invention se présentent avantageusement dans une phase huileuse avec laquelle il constitue un gel anhydre. Ce gel peut être notamment obtenu comme suit :
- (a) mélange du premier organopolysiloxane (i) et du second organopolysiloxane (ii) ;
- (b) ajout de la phase huileuse au mélange de l'étape (a) ; et
- (c) polymérisation du premier organopolysiloxane (i) et du second organopolysiloxane (ii) en phase huileuse en présence d'un catalyseur de platine.

La phase huileuse utilisée lors de la fabrication du gel anhydre contient une ou plusieurs huiles liquides à températures ambiante (25°C) choisies parmi les huiles hydrocarbonées (c'est-à-dire ne comportant que des carbones et des hydrogènes) et/ou les huiles de silicone. Avantageusement, la phase huileuse est une phase liquide siliconée, contenant une ou plusieurs huiles choisies parmi les polydiméthylsiloxanes (PDMS) à chaîne linéaire ou cyclique, liquides à température ambiante comportant éventuellement une chaîne alkyle ou aryle pendante ou en bout de chaîne, la chaîne alkyle ayant de 1 à 6 atomes de carbone.

Les organopolysiloxanes de l'invention sont en particulier obtenus selon le mode opératoire des exemples 3, 4 et 8 du document US-A-5,412,004 et des exemples du document US-A-5,811,487.

Les organopolysiloxanes de la composition de l'invention sont par exemple celui commercialisé sous la référence KSG 21 par la société Shin Etsu ou le produit de l'exemple 3 (exemple de synthèse) du brevet US-A-5,412,004.

Le KSG 21 se présente sous forme d'un gel pâteux comprenant environ 28 % d'organopolysiloxane et 72 % d'huile de silicone (PDMS) ayant une viscosité de 6 cSt (soit 6.10⁻⁶ m²/s).

Selon un mode préféré de réalisation de l'invention, on utilise le produit de l'exemple 3 (exemple de synthèse) du brevet US-A-5,412,004. Ce produit se présente sous la forme d'un gel pâteux contenant environ 33 % en poids d'organopolysiloxane réticulé à groupement(s) oxyéthyléné(s) et environ 67 % de PDMS 6 cSt (soit 6.10⁻⁶ m²/s). L'organopolysiloxane contient environ 18 % d'oxyde d'éthylène en poids par rapport au poids total du polymère.

Le gel élastomère utilisé dans la composition de l'invention a un comportement rhéologique plastique, présentant une viscosité sous faible cisaillement voisin de 10⁻³ s⁻¹ ou 10⁻⁴ s⁻¹, allant de 2.10⁶ Poises à 4.10⁶ Poises (2.10⁵ Pa.s à 4.10⁵ Pa.s), et une viscosité dynamique de 15 à 50 Poises (1,5 à 5 Pa.s) pour une vitesse de cisaillement de 200 s⁻¹ à t_{10 minutes}, mesurée avec un rhéomètre à contrainte imposée, RS 75 (Haake) à 25°C en géométrie cône/plan ; caractéristique du cône : 20 mm de diamètre, 1° d'angle et 40 µm de gap. Cet organopolysiloxane a, en outre, un comportement viscoélastique avec un caractère élastique dominant aux faibles valeurs de la contrainte de cisaillement, défini comme suit : 800 Pa < G*ₚₗₐₜₑₐᵤ < 2.500 Pa avec δ ₚₗₐₜₑₐᵤ voisin de 10°, G*ₚₗₐₜₑₐᵤ représentant le module de rigidité (ou module complexe) c'est-à-dire la consistance, et étant mesuré à 1 Hz, et δ ₚₗₐₜₑₐᵤ représentant l'élasticité (ou angle de perte). Il présente un point éclair d'environ 170°C à la pression atmosphérique.

Pour le produit de l'exemple 3 (exemple de synthèse) du document US-A-5,412,004, la viscosité dynamique, dans les conditions indiquées ci-dessus, est de 45 Poises (4,5 Pa.s).

De façon préférentielle, le gel d'organopolysiloxane élastomère utilisé dans la composition de l'invention est présent dans la composition en une teneur allant de 0,5 à 99 % en poids, et mieux de 3 à 75 % en poids par rapport au poids total de la composition, ce qui correspond à un taux d'organopolysiloxane élastomère en matière active de 0,1 à 33 % en poids et mieux de 1 à 25 % en poids par rapport au poids total de la composition.

En particulier, les particules d'organopolysiloxane élastomère (en matière active) ont une taille allant de 0,1 à 500 µm, de préférence de 3 à 200 µm et mieux de 3 à 50 µm. Ces particules peuvent être sphériques, plates ou amorphes avec, de préférence, une forme sphérique.

L'organopolysiloxane élastomère de l'invention est en particulier un tensioactif de HLB (Balance Hydrophile Lipophile) d'environ 2,5. Il est donc adapté à la fabrication d'émulsions eau-dans-huile. Il peut également permettre, en association avec un autre tensioactif approprié, la préparation d'émulsions huile-dans-eau.

A ce gel d'organopolysiloxane élastomère peut être associée une phase huileuse additionnelle qui peut comprendre des corps gras liquides à la température ambiante, des cires ou des gommes solides à température ambiante, des corps gras pâteux, d'origine animale, végétale, minérale ou synthétique et leurs mélanges.

La phase huileuse additionnelle peut être de toute nature et contenir des produits fluides à température ambiante comme les huiles de silicone, fluorées, fluorosiliconées, hydrocarbonées éventuellement partiellement siliconées. Ces huiles peuvent être volatiles à température ambiante et à la pression atmosphérique. Par huile volatile, on entend en particulier une huile susceptible de s'évaporer, en moins d'une heure, au contact de la peau ou des lèvres, ayant notamment une pression vapeur non nulle, en particulier allant de 10⁻³ à 300 mm de Hg (à température ambiante et pression atmosphérique) et de préférence supérieure à 0,3 mm de Hg.

La quantité de corps gras dans la composition de l'invention peut aller de 1 à 80 % en poids, de préférence de 1 à 50 % et mieux de 1 à 30 % en poids par rapport au poids total de la composition.

Comme huiles utilisables dans la composition de l'invention, on peut citer notamment :
- les huiles hydrocarbonées d'origine animale, telles que le perhydrosqualène ;
- les huiles hydrocarbonées d'origine végétale, telles que les triglycérides liquides d'acides gras, par exemple les huiles de tournesol, de mais, de soja, de courge, de pépins de raisin, de sésame, de noisette, d'abricot, de macadamia, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel ;
- les huiles de formule R¹COOR² dans laquelle R¹ représente le reste d'un acide gras supérieur comportant de 7 à 19 atomes de carbone et R² représente une chaîne hydrocarbonée ramifiée contenant de 3 à 20 atomes de carbone comme par exemple l'huile de Purcellin, le myristate d'isopropyle, des octanoates, décanoates ou ricinoléates d'alcools ou de polyalcools ;
- les hydrocarbures linéaires ou ramifiés, d'origine minérale ou synthétique, tels que les huiles de paraffine volatiles ou non et leurs dérivés, la vaseline, les polydécènes, le polyisobutène hydrogéné tel que le parléam ;
- les éthers de synthèse de formule R³OR⁴ dans laquelle R³ est un radical alkyle en C₃ à C₁₉ et R⁴ un radical alkyle en C₃ à C_{20 ;}
- des alcools gras comme l'octyldodécanol ou l'alcool oléique ;
- les huiles fluorées partiellement hydrocarbonées et/ou siliconées comme les perfluoropolyesters ;
- les huiles de silicone comme les polyméthylsiloxanes à chaîne siliconée linéaire ou cyclique, liquides ou pâteux à température ambiante, les phényldiméthicones, les phényl-triméthicones et les polyméthylphénylsiloxanes, les alkylpolydiméthylsiloxanes avec une chaîne alkyle en C₂ à C₂₀.
- leurs mélanges.

Le gel d'organopolysiloxane à groupement(s) oxyéthyléné(s) permet de structurer ces huiles sous forme d'une texture originale type « flan », exempte de gélifiant huileux qui entraverait le toucher doux/soyeux et agréable de la composition.

La composition de l'invention permet de stabiliser tout actif instable en milieu oxydant, et on peut citer notamment comme actifs instables en milieu oxydant, les vitamines et notamment l'acide ascorbique (vitamine C) et ses dérivés, notamment ses dérivés glycosylés et phosphatés, et ses esters comme l'acétate, le palmitate et le propionate d'ascorbyle, le rétinol (vitamine A) et ses dérivés, notamment ses esters comme l'acétate, le palmitate et le propionate de rétinol ; l'urée ; la rutine ; les enzymes telles que la lipase, la protéase, la phospholipase et les cellulases ; les extraits naturels tels que le thé vert, l'extrait de mélisse, l'extrait de thym, les oligomères procyannidoliques (OPC) tels que l'OPC d'aubépine, l'OPC de pin et l'OPC de raisin ; certains acides tels que l'acide kojique, l'acide caféique, l'acide rétinoique et ses dérivés, l'acide benzène 1,4-di-(3-méthylidène 10-camphosulfonique) ; les caroténoïdes tels que les carotènes comme par exemple les α-, β- et γ-carotènes, le β,ϕ-carotène, le ξ-carotène, le β,λ-carotène, le lycopène (ψ,ψ-carotène) ; les acides gras polyinsaturés tels que l'acide gamma-linolénique, et leurs mélanges.

Il peut s'agir également de tous les composés naturels ou synthétiques pouvant contenir les actifs indiqués ci-dessus, en particulier les extraits végétaux, et plus spécialement les extraits de fruits.

La composition de l'invention est particulièrement intéressante pour stabiliser les vitamines, notamment la vitamine C et la vitamine A, et les caroténoïdes, notamment le lycopène.

La quantité d'actif instable en milieu oxydant dans la composition selon l'invention dépend du type d'actif utilisé et du but recherché. De manière générale, le ou les actifs peuvent être utilisés dans la composition selon l'invention en une quantité allant de 0,01 à 20 % en poids, de préférence de 0,04 à 15 %, et mieux de 0,1 à 10 % en poids par rapport au poids total de la composition.

Selon le caractère hydrophile ou lipophile des actifs utilisés, ceux-ci sont introduits dans la phase huileuse de la composition ou dans une phase aqueuse ajoutée à la composition. Cette dernière peut donc se présenter sous forme d'un produit anhydre ou sous forme d'une émulsion huile-dans-eau ou eau-dans-huile ou d'une émulsion multiple autre qu'une E/H/E ou H/E/H. On entend ici par "émulsion" aussi bien des dispersions sans émulsionnants que des dispersions comportant des émulsionnants ou encore des dispersions stabilisées par des particules solides ou par des sphérules lipidiques de type ionique ou non ionique. Ainsi, le rétinol qui est lipophile peut être introduit dans une composition totalement anhydre tandis que l'acide ascorbique qui est hydrophile est de préférence introduit dans la phase aqueuse d'une émulsion.

Par ailleurs, la composition de l'invention peut être plus ou moins fluide et avoir l'aspect d'une lotion, d'un gel, d'une crème, d'un produit coulé et même se présenter sous forme d'aérosol.

Quand la composition selon l'invention est anhydre, la phase huileuse, en incluant la quantité du ou des organopolysiloxanes élastomères, est présente en une concentration allant de 80 à 99,95 % et de préférence de 90 à 99,9 % du poids total de la composition.

Dans les compositions de l'invention sous forme d'émulsion, la phase aqueuse de la composition est présente en une concentration allant de 1 à 75 %, de préférence 20 à 70 % et mieux de 40 à 70 % du poids total de la composition, et la phase huileuse en incluant la quantité du ou des organopolysiloxanes élastomères, est présente en une concentration allant de 25 à 99 %, de préférence de 30 à 80 % et mieux de 30 à 60 % du poids total de la composition.

Avantageusement, quand la composition de l'invention est une émulsion E/H, elle est exempte de tensioactif autre que l'organopolysiloxane élastomère utilisé selon l'invention. Toutefois, elle peut éventuellement contenir un tensioactif de préférence siliconé, tel que par exemple les mélanges dimethicone copolyol/cyclométhicone (10/90), vendus sous les dénominations « DC-3225 C » et « DC-5225C » par la société Dow Corning.

Quand elle est sous forme d'émulsion H/E, la composition de l'invention contient de préférence au moins un autre émulsionnant. Comme émulsionnant des émulsions H/E, on peut citer par exempte le polydiméthyl méthylsiloxane oxyéthyléné (diméthicone copolyol) vendu sous la dénomination "DC2-5695" par la société DOW CORNING.

Le ou les émulsionnants peuvent être présents dans la composition selon l'invention, en une concentration qui peut varier dans une large mesure. Ainsi, cette concentration peut aller par exemple de 0,1 à 20 %, et de préférence de 1 à 5 % en poids par rapport au poids total de la composition.

Pour augmenter encore la stabilité des actifs instables en milieu oxydant, la composition de l'invention peut comprendre aussi de manière avantageuse au moins un composé choisi parmi les agents séquestrant les métaux, les métabisulfites et les polyols.

L'agent séquestrant les métaux peut être notamment un dérivé d'acide phosphonique. Comme dérivés d'acide phosphonique utilisables dans la composition de l'invention, on peut citer en particulier l'acide éthylènediamine tétra(méthylène phosphonique), l'acide hexaméthylènediamine tétra(méthylène phosphonique), l'acide diéthylènetriamine penta(méthylènephosphonique), et leurs sels et notamment leur sels de sodium comme le sel pentasodique de l'acide éthylènediamine tétra(méthytène phosphonique). De manière avantageuse, on utilise l'acide éthylènediamine tétra(méthylène phosphonique), notamment vendu par la Société MONSANTO sous la dénomination Dequest 2041 et le sel pentasodique de cet acide, vendu sous la dénomination Dequest 2046 par la Société MONSANTO.

Lorsqu'il est présent, l'agent séquestrant est en une concentration allant en général de 0,05 à 0,5 % en poids par rapport au poids total de la composition.

Le métabisulfite peut être un sel alcalin, alcalino-terreux ou d'ammonium de l'acide anhydrosulfureux. On utilise de préférence le métabisulfite de sodium ou de potassium. Lorsqu'il est présent, le métabisulfite est en une concentration allant en général de 0,005 à 5 % et, de préférence, de 0,05 à 1 % en poids par rapport au poids total de la composition.

Les polyols éventuellement présents dans la composition de l'invention peuvent, par exemple, être choisis parmi la glycérine, les glycols tels que le propylène glycol et le polyéthylène glycol, et les silicones comportant des groupes hydroxyle. Les polyols sont présents en une concentration allant, de préférence, de 0,5 à 30 % et préférentiellement de 5 à 25 % du poids total de la composition.

La composition de l'invention est notamment utilisable en application topique, en particulier dans les domaines cosmétique et dermatologique. Cette composition peut être plus ou moins fluide et avoir l'aspect d'une crème blanche ou colorée, d'une pommade, d'un lait, d'une lotion, d'un sérum, d'une pâte, d'une mousse. La composition de l'invention peut être appliquée par voie topique, sur le visage, y compris autour des yeux, sur le corps ainsi que sur le cuir chevelu des êtres humains.

De façon connue, la composition de l'invention peut contenir également des additifs habituels dans les domaines cosmétique et dermatologique, tels que les actifs hydrophiles ou lipophiles autres que les actifs instables en milieu oxydant, les conservateurs, les antioxydants, les solvants, les charges, les absorbeurs d'odeur et les matières colorantes, dans la mesure où l'additif ne déstabilise pas l'actif présent dans la composition. Les quantités de ces différents additifs sont celles classiquement utilisées dans les domaines considérés, et par exemple de 0,01 à 20 % du poids total de la composition. Ces additifs, selon leur nature, peuvent être introduits dans la phase huileuse ou dans la phase aqueuse.

Comme gélifiants hydrophiles, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles, et comme gélifiants lipophiles, on peut citer les argiles modifiées comme les bentones, les sels métalliques d'acides gras, la silice hydrophobe et les polyéthylènes. On peut utiliser notamment comme gélifiants le produit vendu sous le nom de SEPIGEL 305 par la société SEPPIC (nom CTFA : polyacrylamide/C13-14 isoparaffin/Laureth-7) et le produit vendu sous le nom de HOSTACERIN AMPS par la société HOECHST (nom CTFA : Ammonium polyacryldimethyltauramide).

La composition de l'invention peut être utilisée dans toutes les applications des actifs instables décrits ci-dessus, et notamment pour nettoyer la peau et/ou pour la traiter, en particulier pour la tonifier, la régénérer, pour traiter les rides et/ou les ridules de la peau, pour éclaircir le teint, pour éliminer les taches pigmentaires de la peau, pour lutter contre les méfaits des rayonnements UV et/ou pour renforcer les tissus cutanés contre les agressions de l'environnement.

Aussi, la présente invention a aussi pour objet l'utilisation cosmétique de la composition selon l'invention pour nettoyer la peau et/ou pour traiter la peau et en particulier pour la tonifier, la régénérer, pour traiter les rides et/ou les ridules de la peau, pour éclaircir le teint, pour éliminer les taches pigmentaires de la peau, pour lutter contre les méfaits des rayonnements UV et/ou pour renforcer les tissus cutanés contre les agressions de l'environnement.

L'invention a enfin pour objet un procédé cosmétique de nettoyage et/ou de traitement de la peau, qui consiste à appliquer sur la peau, y compris autour des yeux, une composition conforme à l'invention.

L'invention a aussi pour objet l'utilisation de la composition selon l'invention pour la fabrication d'une crème destinée à un traitement de la peau.

D'autres avantages et caractéristiques de l'invention apparaîtront mieux à la lecture des exemples donnés à titre illustratif et non limitatif. Les quantités y sont données en % en poids, sauf mention contraire.

### Exemple 1 : Crème pour le visage (émulsion E/H)

### Phase huileuse :

- Cyclométhicone 10 %
- Phényltriméthicone (Dow Corning 556 Fluid) 4 %
- Huile d'abricot 3 %
- Silicone modifiée (exemple 3 du brevet US-5,412,004) 20 %
(soit 6,6 % de matière active)

### Phase aqueuse:

- Glycérine 23 %
- Propylène glycol 6 %
- Hydroxyde de sodium 1,8 %
- Acide citrique 1,2 %
- Acide ascorbique 5 %
- Eau permutée qsp 100 %

L'émulsion est préparée par préparation des phases et introduction de la phase aqueuse dans la phase huileuse sous agitation à froid.

La composition obtenue se présente sous forme d'une crème appropriée pour le soin du visage, douce à l'application. Cette crème procure un éclat du teint immédiat et permet un lissage des imperfections.

Il a été observé une bonne stabilité de l'acide ascorbique dans l'émulsion et une bonne stabilité de l'émulsion elle-même.

### Exemple 2 : Crème pour le visage (émulsion E/H)

### Phase huileuse :

- Dimethicone copolyol / Cyclométhicone (10/90) (DC-5225C) 20 %
- Phényltriméthicone (Dow Coming 556 Fluid) 4 %
- Huile d'abricot 3 %
- Nylon-12 5 %
- Silicone modifiée (KSG 21 à 28 % de matière active) 5 %
(soit 1,4 % de matière active)

### Phase aqueuse:

- Glycérine 23 %
- EDTA disodique 0,05 %
- Propylène glycol 4 %
- Hydroxyde de sodium 1,8 %
- Acide citrique 1,2 %
- Acide ascorbique 5 %
- Eau permutée qsp 100 %

Mode opératoire : on prépare la phase huileuse, on mélange les constituants de la phase aqueuse en ajoutant en dernier le nylon, puis on émulsionne en versant la phase aqueuse dans la phase huileuse sous agitation.

La composition obtenue se présente sous forme d'une crème appropriée pour le soin du visage, douce à l'application. Cette crème procure un éclat du teint immédiat et permet un lissage des imperfections.

Il a été observé une bonne stabilité de l'acide ascorbique dans l'émulsion et une bonne stabilité de l'émulsion elle-même, même après centrifugation.

**Exemple comparatif :** la même émulsion que celle de l'exemple 2, mais ne contenant pas de KSG-21 se sépare en deux phases et se destabilise après centrifugation.

### Exemple 3 : Crème pour le visage (émulsion E/H)

### Phase huileuse :

- Dimethicone copolyol / Cyclométhicone (10/90) (DC-5225C) 20 %
- Phényltriméthicone (Dow Corning 556 Fluid) 4 %
- Huile d'abricot 3 %
- Isononanoate d'octyle 1,2 %
- Nylon-12 5 %
- Silicone modifiée (KSG 21 à 28 % de matière active) 3,75 %
(soit 1,4 % de matière active)

### Phase aqueuse:

- Glycérine 23 %
- EDTA disodique 0,05 %
- Propylène glycol 4 %
- Hydroxyde de sodium 1,8 %
- Acide citrique 1,2 %
- Acide ascorbique 5 %
- Eau permutée qsp 100 %

L'émulsion est préparée selon le même procédé que dans l'exemple 2.

L'émulsion obtenue est stable, et est apte à procurer l'éclat du teint et à lisser les imperfections de la peau.

## Revendications

1. Composition contenant dans un milieu physiologiquement acceptable, au moins un actif instable en milieu oxydant et une phase huileuse comprenant des particules d'un organopolysiloxane solide élastomère réticulé comportant au moins un groupement oxyalkyléné, la dite composition n'étant pas une émulsion triple H/E/H ou E/H/E.

2. Composition selon la revendication 1, **caractérisée en ce que** l'organopolysiloxane élastomère comporte au moins un groupement oxyéthyléné.

3. Composition selon la revendication 1 ou 2, **caractérisée en ce que** l'organopolysiloxane élastomère ne comporte que des groupements oxyéthylénés comme groupements oxyalkylénés.

4. Composition selon l'une quelconque des revendications précédentes, **caractérisée en ce que** l'organopolysiloxane élastomère est obtenu par réaction d'addition et de réticulation en milieu non aqueux, en présence d'un catalyseur, d'au moins :
- un premier organopolysiloxane (i) ayant au moins deux groupements vinyliques en position α-ω de la chaîne siliconée par molécule ; et
- un second organopolysiloxane (ii) ayant au moins un atome d'hydrogène lié à un atome de silicium par molécule et au moins un groupement oxyalkyléné.

5. Composition selon la revendication précédente, **caractérisée en ce que** le premier organopolysiloxane (i) est choisi parmi les polydiméthylsiloxanes.

6. Composition selon l'une des revendications 4 à 5, **caractérisée en ce que** le premier organopolysiloxane (i) est un α-ω-diméthylvinyl polydiméthylsiloxane.

7. Composition selon l'une des revendications 4 à 6, **caractérisée en ce que** le second organopolysiloxane (ii) est choisi parmi les polydiméthylsiloxanes comportant un ou plusieurs atomes d'hydrogène et un ou plusieurs groupements oxyalkylénés liés à un atome de silicium via un radical alkylène ayant de 1 à 22 atomes de carbone.

8. Composition selon l'une quelconque des revendications précédentes, **caractérisée en ce que** les particules d'organopolysiloxane sont sous forme d'un gel obtenu selon les étapes suivantes :
- (a) mélange du premier et second organopolysiloxanes (i) et (ii) ;
- (b) ajout de la phase huileuse au mélange de l'étape (a) ;
- (c) polymérisation du premier et second organopolysiloxanes (i) et (ii) en phase huileuse en présence d'un catalyseur de platine.

9. Composition selon la revendication précédente, **caractérisée en ce que** le gel a un module de rigidité G* ₚₗₐₜₑₐᵤ mesuré à 1 Hz, défini comme suit : 800 Pa < G* ₚₗₐₜₑₐᵤ < 2 500 Pa avec δ ₚₗₐₜₑₐᵤ voisin de 10°, δ ₚₗₐₜₑₐᵤ représentant l'élasticité.

10. Composition selon l'une quelconque des revendications précédentes, **caractérisée en ce que** les particules d'organosiloxane élastomère ont une taille allant de 3 à 200 µm et de préférence de 3 à 50 µm.

11. Composition selon l'une des revendications précédentes, **caractérisée en ce que** l'organopolysiloxane élastomère représente de 0,1 à 33 % de matière active en poids par rapport au poids total de la composition.

12. Composition selon l'une quelconque des revendications précédentes, **caractérisée en ce que** la phase huileuse contient une ou plusieurs huiles liquides hydrocarbonées et/ou de silicone.

13. Composition selon l'une des revendications précédentes, **caractérisée en ce qu'**elle contient, en outre, au moins un corps gras choisi parmi les huiles volatiles ou non, les cires, les gommes et les corps gras pâteux, d'origine animale, végétale, minérale ou synthétique, et leurs mélanges.

14. Composition selon la revendication précédente, **caractérisée en ce que** la quantité de corps gras va de 1 à 80 % en poids par rapport au poids total de la composition.

15. Composition selon l'une des revendications précédentes, **caractérisée en ce qu'**elle comprend, en outre, une phase aqueuse représentant de 1 à 75 % et mieux de 20 à 70 % du poids total de la composition.

16. Composition selon l'une des revendications précédentes, **caractérisée en ce que** l'actif instable en milieu oxydant est choisi parmi les vitamines, l'urée, la rutine, les enzymes, les caroténoïdes, les extraits naturels, les acides gras polyinsaturés et leurs mélanges.

17. Composition selon l'une des revendications précédentes, **caractérisée en ce que** l'actif instable en milieu oxydant est choisi parmi la vitamine C, la vitamine A, les carotènes et leurs mélanges.

18. Composition selon l'une des revendications précédentes, **caractérisée en ce qu'**elle comprend, en outre, au moins un composé choisi parmi les agents séquestrant les métaux, les métabisulfites et les polyols.

19. Composition selon l'une des revendications précédentes, **caractérisée en ce qu'**elle constitue un produit anhydre ou une émulsion.

20. Utilisation cosmétique de la composition selon l'une quelconque des revendications 1 à 19, pour nettoyer la peau et/ou pour traiter la peau et en particulier pour la tonifier, la régénérer, pour traiter les rides et/ou les ridules de la peau, pour éclaircir le teint, pour éliminer les taches pigmentaires de la peau, pour lutter contre les méfaits des rayonnements UV et/ou pour renforcer les tissus cutanés contre les agressions de l'environnement.

21. Procédé cosmétique de nettoyage et/ou de traitement de la peau, qui consiste à appliquer sur la peau, y compris autour des yeux, une composition selon l'une quelconque des revendications 1 à 19.

22. Utilisation de la composition selon l'une quelconque des revendications 1 à 19, pour la fabrication d'une crème destinée à un traitement de la peau.

23. Utilisation de particules d'organopolysiloxane élastomère solide réticulé comportant au moins un groupement oxyalkyléné, pour la stabilisation d'un actif instable en milieu oxydant.

24. Utilisation selon la revendication précédente, **caractérisée en ce que** les particules d'organopolysiloxane élastomère sont obtenues selon l'une des revendications 4 à 8.

## Patentansprüche

1. Zusammensetzung, die in einem physiologisch akzeptablen Medium mindestens einen in einem oxidierenden Medium instabilen Wirkstoff und eine Ölphase enthält, die Partikel eines vernetzten elastomeren festen Polysiloxans enthält, welches mindestens eine alkoxylierte Gruppe aufweist, wobei die Zusammensetzung keine dreifache O/W/O-Emulsion oder W/O/W-Emulsion ist.

2. Zusammensetzung nach Anspruch 1, **dadurch gekennzeichnet, dass** das elastomere Organopolysiloxan mindestens eine ethoxylierte Gruppe aufweist.

3. Zusammensetzung nach Anspruch 1 oder 2, **dadurch gekennzeichnet, dass** das elastomere Organopolysiloxan als alkoxylierte Gruppen nur ethoxylierte Gruppen enthält.

4. Zusammensetzung nach einem der vorhergehenden Ansprüche, **dadurch gekennzeichnet, dass** das elastomere Organosiloxan in einem nicht wässrigen Medium in Gegenwart eines Katalysators hergestellt wird durch Addition und Vernetzung von zumindest den folgenden Verbindungen:
- einem ersten Organopolysiloxan (i), das mindestens zwei Vinylgruppen in α-ω-Stellung der Siliconkette pro Molekül aufweist; und
- einem zweiten Organopolysiloxan (ii), das pro Molekül mindestens ein Wasserstoffatom, das an ein Siliciumatom gebunden ist, und mindestens eine alkoxylierte Gruppe aufweist.

5. Zusammensetzung nach dem vorhergehenden Anspruch, **dadurch gekennzeichnet, dass** das erste Organopolysiloxan (i) unter den Polydimethylsiloxanen ausgewählt ist.

6. Zusammensetzung nach einem der Ansprüche 4 bis 5, **dadurch gekennzeichnet, dass** das erste Organopolysiloxan (i) ein α-ω-Dimethylvinylpolydimethylsiloxan ist.

7. Zusammensetzung nach einem der Ansprüche 4 bis 6, **dadurch gekennzeichnet, dass** das zweite Organopolysiloxan (ii) unter den Polydimethylsiloxanen ausgewählt ist, die ein oder mehrere Wasserstoffatome und eine oder mehrere Oxyalkylengruppen aufweisen, die über eine Alkylengruppe mit 1 bis 22 Kohlenstoffatomen an ein Siliciumatom gebunden sind.

8. Zusammensetzung nach einem der vorhergehenden Ansprüche, **dadurch gekennzeichnet, dass** die Organopolysiloxanpartikel in Form eines Gels vorliegen, das nach den folgenden Schritten hergestellt wird:
- (a) Mischen des ersten und zweiten Organopolysiloxans (i) und (ii);
- (b) Zugabe der Ölphase zu dem Gemisch aus Schritt (a); und
- (c) Polymerisation des ersten und zweiten Organopolysiloxans
(i) und (ii) in der Ölphase in Gegenwart eines Platinkatalysators.

9. Zusammensetzung nach dem vorhergehenden Anspruch, **dadurch gekennzeichnet, dass** das Gel einen bei 1 Hz gemessenen Schubmodul G*_{Plateau} besitzt, der folgender Definition entspricht: 800 Pa < G*_{Plateau} < 2500 Pa mit δ_{Plateau} ungefähr 10°, wobei δ_{Plateau} die Elastizität bedeutet.

10. Zusammensetzung nach einem der vorhergehenden Ansprüche, **dadurch gekennzeichnet, dass** die Partikel des elastomeren Organopolysiloxans eine Größe von 3 bis 200 µm und vorzugsweise 3 bis 50 µm aufweisen.

11. Zusammensetzung nach einem der vorhergehenden Ansprüche, **dadurch gekennzeichnet, dass** das elastomere Organopolysiloxan 0,1 bis 33 Gew.-% (Wirkstoff), bezogen auf das Gesamtgewicht der Zusammensetzung, ausmacht.

12. Zusammensetzung nach einem der vorhergehenden Ansprüche, **dadurch gekennzeichnet, dass** die Ölphase ein oder mehrere flüssige Kohlenwasserstofföle und/oder Siliconöle enthält.

13. Zusammensetzung nach einem der vorhergehenden Ansprüche, **dadurch gekennzeichnet, dass** sie ferner mindestens eine Fettsubstanz enthält, die unter den flüchtigen oder nicht flüchtigen Ölen, Wachsen, Gummis/Gummen und pastösen Fettsubstanzen tierischer, pflanzlicher, mineralischer oder synthetischer Herkunft oder den Gemischen dieser Verbindungen ausgewählt ist.

14. Zusammensetzung nach dem vorhergehenden Anspruch, **dadurch gekennzeichnet, dass** der Mengenanteil der Fettsubstanz im Bereich von 1 bis 80 Gew.-%, bezogen auf das Gesamtgewicht der Zusammensetzung, liegt.

15. Zusammensetzung nach einem der vorhergehenden Ansprüche, **dadurch gekennzeichnet, dass** sie ferner eine wässrige Phase enthält, die 1 bis 75 % und besser 20 bis 70 % des Gesamtgewichts der Zusammensetzung ausmacht.

16. Zusammensetzung nach einem der vorhergehenden Ansprüche, **dadurch gekennzeichnet, dass** der in einem oxidierenden Medium instabile Wirkstoff unter den Vitaminen, Harnstoff, Rutin, Enzymen, Carotinoiden, natürlichen Extrakten, mehrfach ungesättigten Fettsäuren und deren Gemischen ausgewählt ist.

17. Zusammensetzung nach einem der vorhergehenden Ansprüche, **dadurch gekennzeichnet, dass** der in einem oxidierenden Medium instabile Wirkstoff unter Vitamin C, Vitamin A, Carotinen und deren Gemischen ausgewählt ist.

18. Zusammensetzung nach einem der vorhergehenden Ansprüche, **dadurch gekennzeichnet, dass** sie ferner mindestens eine Verbindung enthält, die unter den Maskierungsmitteln für Metalle, Disulfiten und Polyolen ausgewählt ist.

19. Zusammensetzung nach einem der vorhergehenden Ansprüche, **dadurch gekennzeichnet, dass** sie ein wasserfreies Produkt oder eine Emulsion ist.

20. Kosmetische Verwendung der Zusammensetzung nach einem der Ansprüche 1 bis 19 zur Reinigung der Haut und/ oder zur Behandlung der Haut, insbesondere um die Haut zu straffen, die Haut zu regenerieren, Falten und/oder Fältchen der Haut zu behandeln, den Teint strahlender erscheinen zu lassen, Pigmentflecken der Haut zu entfernen, die schädlichen Wirkungen der UV-Strahlung zu bekämpfen und/oder das Hautgewebe gegenüber Angriffen aus der Umgebung zu stärken.

21. Kosmetisches Verfahren zur Reinigung und/oder zur Behandlung der Haut, das darin besteht, auf die Haut einschließlich der Partie um die Augen eine Zusammensetzung nach einem der Ansprüche 1 bis 19 aufzutragen.

22. Verwendung der Zusammensetzung nach einem der Ansprüche 1 bis 19 zur Herstellung einer Creme, die zur Behandlung der Haut vorgesehen ist.

23. Verwendung von Partikeln eines vernetzten festen elastomeren Organopolysiloxans, das mindestens eine Oxyalkylengruppe aufweist, zur Stabilisierung eines in einem oxidierenden Medium instabilen Wirkstoffs.

24. Verwendung nach dem vorhergehenden Anspruch, **dadurch gekennzeichnet, dass** die Partikel des elastomeren Organopolysiloxans nach einem der Ansprüche 4 bis 8 hergestellt werden.

## Claims

1. Composition comprising, in a physiologically acceptable medium, at least one active principle which is unstable in oxidizing medium and an oily phase comprising particles of a crosslinked solid organopolysiloxane elastomer comprising at least one oxyalkylene group, the said composition not being an O/W/O or W/O/W triple emulsion.

2. Composition according to Claim 1, **characterized in that** the organopolysiloxane elastomer comprises at least one oxyethylene group.

3. Composition according to Claim 1 or 2, **characterized in that** the organopolysiloxane elastomer only comprises oxyethylene groups as oxyalkylene groups.

4. Composition according to any one of the preceding claims, **characterized in that** the organopolysiloxane elastomer is obtained by an addition and crosslinking reaction in a non-aqueous medium, in the presence of a catalyst, of at least:
- one first organopolysiloxane (i) having at least two vinyl groups in the α,ω-position of the silicone chain per molecule; and
- one second organopolysiloxane (ii) having at least one hydrogen atom bonded to a silicon atom per molecule and at least one oxyalkylene group.

5. Composition according to the preceding claim, **characterized in that** the first organopolysiloxane (i) is chosen from polydimethylsiloxanes.

6. Composition according to either of Claims 4 and 5, **characterized in that** the first organopolysiloxane (i) is an α,ω-dimethylvinylpolydimethylsiloxane.

7. Composition according to one of Claims 4 to 6, **characterized in that** the second organopolysiloxane (ii) is chosen from polydimethylsiloxanes comprising one or more hydrogen atoms and one or more oxyalkylene groups bonded to a silicon atom via an alkylene radical having from 1 to 2 2 carbon atoms.

8. Composition according to any one of the preceding claims, **characterized in that** the organopolysiloxane particles are in the form of a gel obtained according to the following stages:
- (a) mixing the first and second organopolysiloxanes (i) and (ii);
- (b) adding the oily phase to the mixture of stage (a);
- (c) polymerizing the first and second organopolysiloxanes (i) and (ii) in the oily phase in the presence of a platinum catalyst.

9. Composition according to the preceding claim, **characterized in that** the gel has a stiffness modulus G*ₚₗₐₜₑₐᵤ measured at 1Hz which is defined as follows : 800 Pa < G*ₚₗₐₜₑₐᵤ < 2500 Pa with δₚₗₐₜₑₐᵤ in the region o f 10°, δₚₗₐₜₑₐᵤ representing the elasticity.

10. Composition according to any one of the preceding claims, **characterized in that** the elastomeric organosiloxane particles have a size ranging from 3 to 200 µm and preferably from 3 to 50 µm.

11. Composition according to one of the preceding claims, **characterized in that** the organopolysiloxane elastomer represents from 0.1 to 33% of active material by weight with respect to the total weight of the composition.

12. Composition according to any one of the preceding claims, **characterized in that** the oily phase comprises one or more liquid hydrocarbonaceous and/or silicone oils.

13. Composition according to one of the preceding claims, **characterized in that** it additionally comprises at least one fatty substance chosen from volatile and non-volatile oils or from waxes, gums and pasty fatty substances of animal, vegetable, mineral or synthetic origin, and their mixtures.

14. Composition according to the preceding claim, **characterized in that** the amount of fatty substance ranges from 1 to 80% by weight with respect to the total weight of the composition.

15. Composition according to one of the preceding claims, **characterized in that** it additionally comprises an aqueous phase representing from 1 to 75% and better still from 20 to 70% of the total weight of the composition.

16. Composition according to one of the preceding claims, **characterized in that** the active principle which is unstable in oxidizing medium is chosen from vitamins, urea, rutin, enzymes, carotenoids, natural extracts, polyunsaturated fatty acids and their mixtures.

17. Composition according to one of the preceding claims, **characterized in that** the active principle which is unstable in oxidizing medium is chosen from vitamin C, vitamin A, carotenes and their mixtures.

18. Composition according to one of the preceding claims, **characterized in that** it additionally comprises at least one compound chosen from metal-sequestering agents, metabisulphites and polyols.

19. Composition according to one of the preceding claims, **characterized in that** it constitutes an anhydrous product or an emulsion.

20. Cosmetic use of the composition according to any one of Claims 1 to 19 for cleansing the skin and/or for treating the skin and in particular for toning it up, regenerating it, for treating wrinkles and/or fine lines of the skin, for improving the complexion, for removing pigmentary blemishes of the skin, for combating damage due to UV radiation and/or for strengthening cutaneous tissues against attacks from the environment.

21. Cosmetic process for cleansing and/or treating the skin which consists in applying, to the skin, including around the eyes, a composition according to any one of Claims 1 to 19.

22. Use of the composition according to any one of the Claims 1 to 19 in the manufacture of a cream intended for a treatment of the skin.

23. Use of particles of crosslinked solid organopolysiloxane elastomer comprising at least one oxyalkylene group for the stabilization of an active principle which is unstable in oxidizing medium.

24. Use according to the preceding claim, **characterized in that** the particles of organopolysiloxane elastomer are obtained according to one of Claims 4 to 8.
